(19)

Europäisches Patentamt

European Patent Office

Office européen des brevets

(11) **EP 4 591 814 A1**

(12) **EUROPEAN PATENT APPLICATION**

(43) Date of publication:
**30.07.2025 Bulletin 2025/31**

(21) Application number: **24153333.0**

(22) Date of filing: **23.01.2024**

(51) International Patent Classification (IPC):
**A61B 18/12** (2006.01)    **A61B 18/14** (2006.01)
**A61B 18/00** (2006.01)

(52) Cooperative Patent Classification (CPC):
**A61B 18/1206; A61B 18/1233; A61B 18/1492;**
A61B 2018/0016; A61B 2018/00178;
A61B 2018/00375; A61B 2018/00613;
A61B 2018/00642; A61B 2018/00755;
A61B 2018/00875; A61B 2018/1467

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **VascoMed GmbH**
**79589 Binzen (DE)**

(72) Inventors:
• **Rehberger, Frank**
  **79111 Freiburg (DE)**
• **Holzinger, Steffen**
  **12437 Berlin (DE)**

(74) Representative: **Biotronik Corporate Services SE**
**Corporate Intellectual Property**
**Sieversufer 7-9**
**12359 Berlin (DE)**

(54) **ABLATION CATHETER AND SYSTEM COMPRISING SUCH CATHETER**

(57)    The invention describes a catheter (10) for treatment of a patient's tissue using an electrical field with at least one ablation electrode (15), each electrode having an ablation electrode input, wherein the catheter (10) further comprises a catheter connector (19) for electrical connection to a generator output of a power supply generator (30). To further improve ablation treatment of the patient's tissue using an electrical field in terms of treatment cost and complexity while ensuring consistent treatment quality, the catheter (10) comprises at least one assembly (20). The assembly (20) comprises a capacitor unit, an assembly input and an assembly output, wherein the capacitor unit comprises at least one capacitor (24) having a predefined capacity and is electrically connected to the assembly input (21) and the assembly output (22), wherein the assembly (20) is configured to be interposed between and electrically connected to the generator output and the ablation electrode input of a predefined ablation electrode (15, 15'). Further, each assembly (20) is formed integrally with the catheter (10) such that the respective assembly input (21) is electrically connected to the catheter connector (19) and the respective assembly output (22) is electrically connected to the ablation electrode input of a predefined electrode (15). A coupling device containing such assembly and a system comprising the ablation catheter and the power supply generator as well as the coupling device is also described.

FIG. 1

**Description**

The invention is directed to an ablation catheter for treatment of a patient's tissue and a system comprising a power supply generator for such ablation catheter and the ablation catheter. In particular, the present invention relates to an ablation catheter that may be used for safely performing cardiac ablation procedures, such as, but not limited to, pulmonary vein isolation (PVI), persistent atrial fibrillation ablation, or ventricular tachycardiac ablation.

It is known to use ablation catheters in the therapy of atrial fibrillation (AF) patients. AF which may show as tachycardia or cardiac irregularity and may, especially if unnoticed, cause stroke, is often treated by electrically isolating the pulmonary vein (PV) from the left atrium by creating contiguous circumferential ablation lesions around the pulmonary vein ostium (PVO) or around their antrum. Thus, circular type ablation (also called circumferential type ablation) of the patient's tissue may be used to avoid irregular atrial contractions by hindering undesired perturbing electrical signals generated within the PV from propagating into the left atrium. Alternatively or additionally, ablation of small local areas or points of tissue may be provided. For example, such type of ablation may be necessary if undesired perturbing electrical signals are generated in a small (focal) area, which spread out from this area in a circular pattern. Even though, ablation catheters may often be used to treat tissue of the left atrium or the PV, the ablation therapy is not limited to these regions and may also be provided to ventricles, right atrium, other organs such as lungs, liver, kidneys, etc.

Thermal energy sources, e.g. radiofrequency or cryoablation, may be used for interventional atrial fibrillation treatment, for example, in the context of pulmonary vein isolation. Such type of ablation, however, is known to destroy not only myocytes but also adjacent tissue.

Pulsed field ablation (PFA) is a form of energy that does not utilize thermal effects. By means of an electrical pulse lasting only fractions of seconds, an irreversible change in cell membrane pores occurs (irreversible electroporation). The myocardium is very sensitive to this form of energy delivery compared to the esophagus, pulmonary veins or phrenic nerve. Consecutively, it is possible to perform effective ablation of the pulmonary veins using PFA in a very short time without injuring adjacent tissue.

To provide a patient specific ablation treatment, different catheter types may be used. Additionally, the treated tissue of the patient may have different electrical properties depending on the tissue type, its composition and thickness and the type of ablation treatment. Accordingly, it may be necessary to keep a variety of different catheters on hand to respond to different patient treatment needs while ensuring consistent treatment quality. Such approach is very cost-intensive and complex during treatment.

It is desirable to further improve ablation treatment of the patient's tissue using an electrical field in terms of treatment cost and complexity while ensuring consistent treatment quality.

The above object is solved by an assembly having the features of claim 1, a catheter having the features of claim 7, a coupling device having the features of claim 10 and a system comprising a catheter and a power supply generator having the features of claim 12.

In particular, the above problem is solved by an assembly for a system comprising a power supply generator having a generator output and a catheter for treatment of a patient's tissue using an electrical field with at least one ablation electrode, each ablation electrode having an ablation electrode input, wherein the assembly comprises a capacitor unit, an assembly input and an assembly output, wherein the capacitor unit comprises at least one capacitor having a predefined capacity and is electrically connected to the assembly input and the assembly output, wherein the assembly is configured to be interposed between and electrically connected to the generator output and the ablation electrode input of a predefined ablation electrode.

The catheter may comprise one ablation electrode or at least two ablation electrodes, wherein in case the catheter carries exactly one ablation electrode a reference/return/grounding electrode may be provided to close the output circuit of the catheter/electrode in order to provide unipolar ablation. The catheter may comprise further electrodes not used for ablation, for example, at least one measuring electrode, for ablation monitoring and/or mapping. The catheter may comprise electrode pairs in order to provide bipolar ablation. Each ablation electrode may comprise an ablation electrode input configured to receive an electrical ablation pulse or electrical ablation pulse train for application to the patient's tissue. The ablation electrode may be formed as a ring electrode or a tip electrode. The input of each ablation electrode is electrically connected to a lead that may be connected by a respective connector (plug or socket) to the power supply generator.

The catheter may comprise a handle at its proximal end, a distal tip at its distal end and an elongated catheter shaft between the handle and the distal tip. The distal end section of the catheter shaft may comprise a functional section comprising the at least one ablation electrode and, if applicable, at least one measuring/mapping electrode. The distal tip of the catheter may form the distal end of the distal end section/functional section.

The handle allows the health care practitioner (HCP) to advance the catheter within the patient's body and/or retract it from the target location of the patient's body, e.g. the patient's vascular system, by pushing and/or pulling. The target location is the patient's tissue area where the ablation is performed. The handle may further comprise an actuating unit for manipulating/steering the distal end of the elongated catheter shaft to easily and precisely apply the at least one electrode to the patient's target location, e.g. by a steering wire.

Each one of the at least one ablation electrode or electrode pair is configured to provide ablation pulses or ablation pulse trains thereby providing PFA or RF ablation as explained below in detail.

The power supply generator provides the power supply to the catheter and its ablation electrodes in the form of pulses or pulse trains configured to be transmitted to the at least one ablation electrode for ablation. To provide the pulses or pulse trains, the generator comprises a pulse-shaping output stage for coupling to the at least one ablation electrode.

The power supply generator provides the pulses or pulse trains provided for ablation by the catheter at its generator output, wherein the shape of each pulse may be a rectangular pulse but also any other type of pulses such as a gaussian pulse, a cosine squared pulse, a Dirac pulse, or a sine pulse may be used. The generator output may comprise one output line or several output lines, wherein one output line may be configured to be connected to one predefined ablation electrode thereby providing the ablation pulses or pulse trains to this predefined ablation electrode. The generator comprises a connector (plug/socket) that mechanically fits in the catheter's connector thereby providing the predefined electrical connection of the generator output (line) and the ablation electrode input of the predefined ablation electrode of the ablation catheter.

According to the invention, an assembly is provided that comprises a capacitor unit, an assembly input and an assembly output, wherein the capacitor unit comprises at least one capacitor having a predefined capacity and is electrically connected to the assembly input and the assembly output. Further, the assembly input is configured to be electrically connected to the generator output so that the generator output is electrically connected via the assembly to the ablation electrode input. The assembly output is configured to be electrically connected to the ablation electrode input and may be mechanically connected to the ablation electrode input fixedly or detachably. Further, in one embodiment, the assembly may be mechanically detachably connected to the generator output.

HCPs aim to work with a consistent pulse shape of ablation pulses for similar treatments. In pursuing this goal, the inventors have observed that the form of the ablation pulse significantly depends on the electrical load associated with the respective ablation electrode and the tissue impedance provided by the specific target location to which the ablation pulses are provided. A change in tissue impedance causes ablation pulse / signal distortion from a desired ablation pulse shape. Further, the inventors have assumed that the electrical circuit is regarded as RC element and have derived from above finding that the distortion may be compensated by introducing a capacitor unit providing a predefined capacity into the electrical circuit. This capacity is provided by at least one capacitor. As indicated below in detail, the compensating capacity may be calculated. According to the invention, the calculated compensating capacity is provided and introduced in the electrical ablation circuit by the above assembly comprising a capacitor unit realizing this capacity thereby reducing the signal distortion and signal shape is changed so that it approximately corresponds to the desired shape. By using such an assembly, either adjustable capacity can be provided or different assemblies can be used for different ablation/patient's tissue conditions to ensure consistent treatment quality.

In one embodiment, for electrical load values of the tissue in the $15\,\Omega$ to $25\,\Omega$ range, desired capacitance values may be in the range of $0.2\,\mu F$ to $0.5\,\mu F$. For loads in the $25\,\Omega$ to $50\,\Omega$ range, desired capacitance values may in the range of $0.1\,\mu F$ to $0.2\,\mu F$. Yet for loads in the $50\,\Omega$ to $150\,\Omega$ range, desired capacitance values may be less than $0.1\,\mu F$. Ideally, if load values vary depending on the number of selected ablation electrodes, ablation configuration, i.e. unipolar or bipolar, or tissue conditions, the capacity of the capacitor unit is adjusted such that the equivalent time constant of the ablation pulse stays in the same range.

Accordingly, by the inventive assembly, an easy and cost-effective way is provided to improve ablation treatment. When using the above assembly, different electrical tissue properties in connection with ablation at different patients and/or different areas within the body of the same patient may be compensated and the pulse/signal form may approximately be maintained. Analogously, the power supply generator and/or the pulse/signal provided by the generator does not need to be changed or exchanged.

A further advantage of the assembly and its usage consists in that it provides a further decoupling from the high DC voltages used within the power supply generator. This increases patient safety and avoids undesired gas generation within the organism due to electrolysis of blood that may be caused by minimal residual DC voltages. Accordingly, microbubbling and thereby embolic events may be avoided.

Further, the capacity provided by the assembly changes the pulse form from a monophasic pulse to a biphasic pulse. Such biphasic pulse is intrinsically a charge-balanced pulse which is highly beneficial as a net charge injection into the stimulated tissue via the respective ablation electrode is prevented. Accordingly, undesired (skeletal) muscle contractions (e.g., which may be caused by direct or indirect stimulation of motor nerves) may be avoided.

Further, the charge-balanced pulse may prevent an electrical arcing caused by ionization of the medium between the respective electrode and the counter electrode.

In one embodiment, the capacitor unit comprises at least two capacitors that are electrically connected in series. In one embodiment, the capacitor unit comprises a parallel connection of at least two capacitors. In one embodiment, the capacitor unit comprises a predefined first number of capacitors connected in series and a predefined second number of capacitors having a parallel electrical connection. The aforementioned capacitor structure allows the ablation pulse shape to be adapted to tissue/catheter/generator conditions over a wide range.

In one embodiment, the at least one capacitor is, for example, a ceramic capacitor, an electrolytic capacitor, a mica capacitor, a film capacitor, wherein, if the capacitor unit comprises more than two capacitors, any combination of above capacitors may be used.

The at least one capacitor of the assembly may be located on a printed circuit board (PCB).

In one embodiment of the assembly, the assembly further comprises an adjustable control element, wherein the control element is configured to control the capacity of an electrically adjustable capacitor according to the setting of the control element, and/or in case the capacitor unit comprises at least two capacitors, the control element is configured to activate a predefined capacitor or predefined capacitors of the at least two capacitors and a predefined interconnection of the activated capacitor(s) according to the setting of the control element. In both embodiments the capacity provided by the assembly/capacitor unit can be adjusted to the needs of the actual ablation treatment. Accordingly, the control element comprises an interface for receiving the desired capacity - i.e. an input means. For example, the input means may be manually operable or an electrical signal may be provided containing the information on the desired compensation capacity that may be transferred to the input means via a wired or wireless connection. The electrically controlled capacitor may be a variable capacitance diode, a BST varactor, tunable RF-MEMS capacitor.

In an embodiment the capacitor unit may be integrated into the handle or a cable connecting the generator and the catheter. In this embodiment the capacitor unit may comprise only one capacitor, in particular an adjustable capacitor connected to the adjustable control element, wherein the control element is configured to control the capacity of an electrically adjustable capacitor according to the setting of the control element.

In addition or alternatively, the capacitor unit may be integrated into a connecting box arranged between the generator and the catheter and thereby electrically connecting the generator and the catheter. In this embodiment, the capacitor unit may comprise at least two, in particular a multitude of, capacitors. The multitude of capacitors is arranged in a way, that a multitude of electrical outputs is formed, whereby the multitude of electrical output is configured for an electrical connection to the catheter.

In one embodiment, the assembly comprises a measuring unit configured to measure the actual impedance of the tissue of the target location. Using this measured impedance, the compensating capacity may be calculated as indicated below in detail. As soon as the impedance is measured, the respective compensating capacity may be calculated and the value transmitted to the control element. The control element may then be automatically set to realize the calculated compensating capacity. Therefore, the assembly may comprise or may be connected to a computing unit that provides the respective data processing using the measured impedance values.

In one embodiment, the assembly comprises a switch unit for switching between at least two ablation modes, wherein the switch unit has a first switch unit input, a second switch unit input and a first switch unit output, wherein the first switch unit output is electrically connected to an input of the capacitor unit, wherein the first switch unit input is electrically connected to the assembly input, wherein the second switch unit input is configured to receive a control signal from the power supply generator or from an adjustment element. The second switch unit input may be electrically connected to the connector and configured to receive a control signal from the power supply generator via the connector of the catheter or wireless, for example, using an antenna that is connected to the second switch unit input. Alternatively, the assembly may comprise an adjustment element, that may be, for example, manually operatable. The at least two ablation modes may comprise the modes "monophasic pulse mode" and "biphasic pulse mode". In the monophasic pulse mode the capacity of the assembly may be reduced, whereas in the biphasic pulse mode the capacity of the assembly may be increased. Additionally or alternatively, the switch unit may realize a PFA ablation mode and an RF ablation mode.

The above problem is further solved by a catheter for treatment of a patient's tissue using an electrical field with at least one ablation electrode, each electrode having an ablation electrode input, wherein the catheter further comprises a catheter connector for electrical connection to a generator output of a power supply generator, wherein the catheter comprises at least one assembly as described above, wherein each assembly is formed integrally with the catheter such that the respective assembly input is electrically connected to the catheter connector and the respective assembly output is electrically connected to the ablation electrode input of a predefined electrode.

In an example, where the catheter comprises at least one assembly as described above, the assembly may comprise a printed circuit board. The printed circuit board may comprise a capacitor unit for each electrode. The individual capacitor units are connected to the individual electrodes, whereby connections to electrodes of opposing polarities may be electrically insulated from each other.

Alternatively, the assembly may comprise capacitor units soldered to the individual electrical connections, in particular within the catheter handle, to the at least one ablation electrodes.

The general properties of the catheter and the power supply generator are described above in connection with the assembly. It is referred to the above explanation, also with regard to the assembly. It is further referred to above properties and advantages of the assembly that are also true for the catheter. In particular, the catheter having the at least one assembly can be adapted to the different patient situations. Accordingly, in one embodiment the provision of different catheter types is not necessary, since the capacity of the at least one assembly is adjustable by the control element.

The catheter is configured to deliver ablation treatment to the patient's tissue using an electrical field by means of at least one ablation electrode. In particular, the ablation catheter may be used to provide cardiac catheter ablation to treat a variety of cardiac arrhythmias including AF. The catheter may also be used for different type of tissue, for example veins, lungs, liver, kidneys, and different therapy, for example to treat tumors. It may be used for pulmonary vein isolation (PVI), persistent atrial fibrillation ablation, ventricular tachycardiac ablation and other ablation procedures.

In one embodiment of the catheter, the catheter comprises a supply line connecting a handle of the catheter and the catheter connector, wherein each assembly is formed integrally with the supply line and/or with the catheter connector. As indicated above, the handle is located at the proximal end of the elongated catheter shaft. From the handle, a supply line may be provided projecting from the handle and comprising a connector at its end opposite from the handle. The supply line electrically connects the electrical contacts of the connector (plug / socket) to the leads of the respective ablation electrodes via the handle. The at least one assembly is located within the connector housing and/or within the supply line thereby covered and electrically isolated by the connector housing or line sheathing such that the assembly input is electrically connected to the respective contact element of the connector and the assembly output is electrically connected to the lead for the respective ablation electrode.

In one embodiment each assembly is formed integrally with the handle of the catheter. Accordingly, the assembly is located within the electrically isolating housing of the connector or of the handle such that the assembly input is electrically connected to the respective contact element of the connector and the assembly output is electrically connected to the lead for the respective ablation electrode.

In one embodiment, the ablation catheter comprises an electronic control unit (ECU) configured to control operation of each of the at least one ablation electrode and, if applicable, each of the at least one additional electrode, wherein the ECU uses a computing processor. The ECU controls the electrodes such that monopolar and/or bipolar ablation as well as PFA or RFA may be provided. A switching device, for example the switch unit of the assembly, which may be a separate or an integral part of the ECU and controlled by the ECU, may be used to switch the electrodes so that desired monopolar and/or bipolar ablation as well as PFA or RFA is realized. The switch unit may comprise a respective switch matrix.

In one embodiment, the operation control of the ECU comprises and/or realizes an ablation mode and a sensing mode with regard to the at least one ablation electrode. This means that the respective electrode may be switched between the sensing mode and the ablation mode, wherein in the sensing mode the respective electrode detects the surrounding electromagnetic signals of the tissue and in the ablation mode the respective electrode provides a pre-defined ablation pulse or pulse train to a target location of a tissue. The ECU may be configured to only switch the mode of all ablation electrodes, a single ablation electrode or of a subgroup of all ablation electrodes at a pre-defined, specific time. In order to ease and improve assessment of the received electromagnetic signals of the electrodes in the sensing mode the ECU may be configured to visualize the signals with regard to their local distribution using standard imaging technology (so-called electro-anatomical mapping).

The above problem is further solved by a coupling device for a system comprising a power supply generator having a generator connector and a catheter for treatment of a patient's tissue using an electrical field with at least one ablation electrode, wherein the catheter comprises a catheter connector, wherein the coupling device comprises an assembly as described above, wherein the assembly is integrally formed with the coupling device, wherein the coupling device is configured to be electrically connected and detachably mechanically connected to the catheter connector and to the generator connector thereby providing an electrical connection from a generator output of the power supply generator to an ablation electrode input via the assembly.

The above problem is further solved by a system comprising a power supply generator having a generator connector and a catheter for treatment of a patient's tissue using an electrical field with at least one ablation electrode, wherein the catheter comprises a catheter connector, wherein the system further comprises a coupling device formed separately from the catheter and from the generator, wherein the coupling device comprises an assembly as described above, wherein the assembly is integrally formed with the coupling device, wherein the coupling device is configured to be electrically connected to the catheter connector and to the generator connector thereby providing an electrical connection from a generator output of the power supply generator to an ablation electrode input via the assembly.

The general properties of the catheter and the power supply generator of the system are described above in connection with the assembly or the catheter. It is referred to the above explanation, also with regard to the assembly. It is further referred to above properties and advantages of the assembly and the catheter that are also true for the system and the coupling device used in the system. In particular, the power supply generator or catheter used with the coupling device having the at least one assembly can be adapted to the different generator/catheter/patient situations. In one embodiment, the provision of different catheter types or alternative power supply generators is not necessary, since the capacity of the at least one assembly of the coupling device is adjustable by the control element.

The coupling device comprises two connectors, a first connector (e.g. plug) that is configured to be mechanically and electrically connected to the respective connector (e.g. socket) of the power supply generator and a second connector (e.g. plug) that is configured to be mechanically and electrically connected to the respective connector (e.g. socket) of the catheter. The connectors may be male or female. In particular, the first connector is configured to electrically connect the assembly input to the power supply generator output and the second connector is configured to electrically connect the assembly output to the ablation electrode input of the respective ablation electrode of the catheter if the first connector is coupled to the power supply generator and/or the second connector is coupled to the catheter.

In one embodiment, the coupling device comprises a box containing the assembly and may include, for example, the control element and/or the measuring unit and/or the switching unit as described above. The box may comprise an interface for providing control signals/information (e.g. for the switching unit and/or the control element) and/or for receiving measuring signals from the measuring unit and/or a display for visualization of the control, switching and/or measurement as indicated above to the health care practitioner (HCP). Alternatively, the assembly may be integrated within an elongated, electrically isolating cable connecting the first and the second connector of the coupling device. The box may be configured such that the first connector and the second connector of the coupling device laterally project from the box. The shape of the box allows easy handling and secure standing and covering of the at least one assembly.

In one embodiment of the system, the generator comprises a pulse-shaping output stage for coupling to the at least one ablation electrode. In one embodiment of the system, the generator comprises a high-voltage source, wherein the high-voltage source is configured to be set by the generator to provide a high-voltage amplitude of at least 1.000 V, preferably at least 1.500 V, more preferably at least 2.000 V, most preferably at least 3.000 V.

The various features and advantages of the present invention may be more readily understood with reference to the following detailed description and the embodiments shown in the drawings. Herein schematically and exemplarily,

Fig. 1 depicts an embodiment of a catheter in a perspective side view and partially cut open,

Fig. 2 shows the handle of the catheter of Fig. 1 in a perspective side view and partially cut open,

Fig. 3 depicts an electrical circuit comprising the catheter of Fig. 1 connected to a power supply generator,

Fig. 4 shows a coupling device of a system according to the invention in a perspective side view and partially cut open,

Fig. 5 depicts one connector of the coupling device of Fig. 4 in a perspective side view and partially cut open,

Fig. 6 depicts an electrical circuit of the system comprising a catheter, the coupling device of Fig. 4 and a power supply generator, and

Fig. 7 the signal shape of the signal prior compensation by the assembly used in the catheter of Fig. 1 or the coupling device of Fig. 4, wherein the x-axis refers to the time and the y-axis to voltage and wherein the upper signal is the generator signal and the lower signal is the pulse provided by a paired ablation electrode, and

Fig. 8 the signal shape of the signal after compensation by the assembly used in the catheter of Fig. 1 or the coupling device of Fig. 4, wherein the x-axis refers to the time and the y-axis to voltage and wherein the upper signal is the generator signal and the lower signal is the pulse provided by a paired ablation electrode.

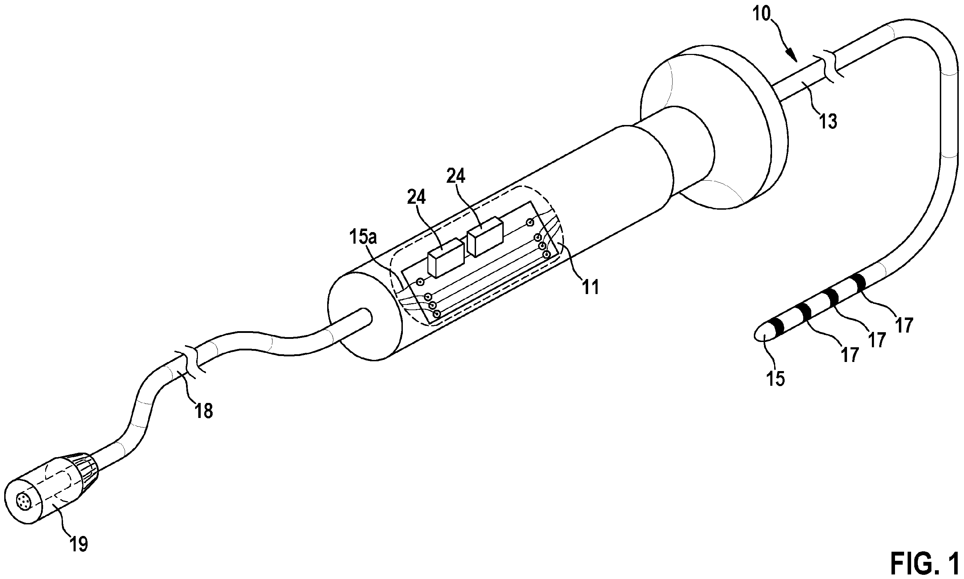

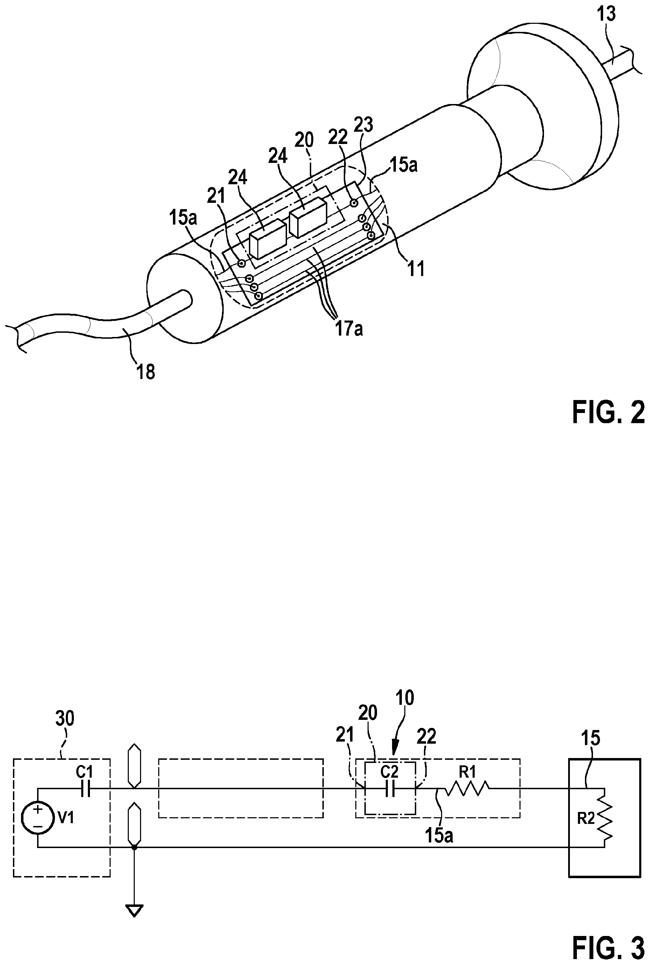

Fig. 1 illustrates an embodiment of an ablation catheter 10. The ablation catheter may be used for PFA, when used with the PFA generator and accessories, and is indicated for use in high-voltage, pulsed-field cardiac ablation. Peak voltages are, for example, without limitation, +/- 1 kV to 3 kV with a pulse width of up to 30 μs, in particular up to 20 μs, in particular up to 5 μs. Higher peak voltages (e.g. up to 10 kV) may be used provided the pulse duration is correspondingly shorter (e.g 0.5 μs). The catheter 10 has a handle 11 that may comprise a steering mechanism, wherein the handle 11 is connected to an elongated catheter shaft 13 that projects from the distal end of the handle 11. At the distal end of the catheter shaft 13 there is a functional section comprising an ablation tip electrode 15 and three ring electrodes 17 accommodated side by side proximal from the tip electrode 15. At the proximal end of the handle 11 a supply line 18 connects a connector (plug) 19 with the handle 11, wherein the connector 19 is configured to be connected with a socket of a power supply generator 30 (not shown in Fig. 1 and 2). The generator 30 provides the power supply and generates the high-voltage ablation pulses having a voltage V1 (see Fig. 3) by means of a pulse generator that are configured to by applied by the tip electrode 15 to the patient's tissue. If the connector 19 is mechanically and electrically coupled to the generator 30, particularly to its generator output, the respective generator output is electrically connected via the lead 15a to the ablation tip electrode 15. As one can derive from the cut out shown in Fig. 2, there are three leads 17a connecting the generator 30 via the connector 19 to the ring electrodes 17.

On a printed circuit board (PCB) 23 accommodated within the housing of the handle 11, an assembly 20 is depicted in Fig. 2 by a dot-dashed line that forms a section of this PCB 23. The assembly 20 comprises an assembly input 21 that is connected via the lead 15a to the connector 19 and an assembly output 22 which is connected via the lead 15a to the ablation tip electrode 15. The assembly 20 comprises two capacitors 24 connected in series and with one end of the first capacitor 24 electrically connected to the assembly input 21 and with the opposite end of the second capacitor 24 to the assembly output 22. Accordingly, the assembly 20 is configured to be interposed between and electrically connected to the generator output and the ablation tip electrode input of the ablation electrode 15.

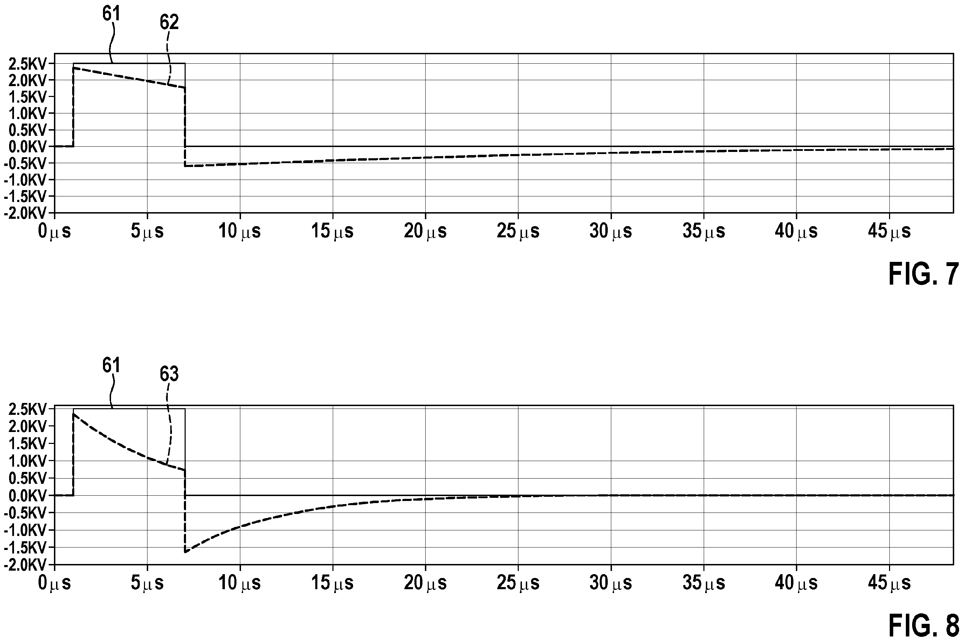

The electrical circuit describing the full system is shown in Fig. 3. The generator 30 comprises a high-voltage power supply providing a voltage V1 and a pulse generator providing, for example rectangular pulses 61 as shown in Fig. 7 and 8. The system is configured to provide a pulse train comprising up to 500 biphasic pulses/train having a pulse width between 0.5 - 30 $\mu$s and a peak voltage up to +/- 10 kV, preferably +/- 1kV-3kV, wherein an internal coupling capacity of the generator 30 is about 200 nF.

A desirable pulse is shown in Fig. 8 having a biphasic exponential decay shape (see curve 63 in Fig. 8) which would be realized in this configuration if the tissue impedance R2 is 20 $\Omega$. However, if the impedance R2 of the tissue is a greater value, for example, R2 = 100 $\Omega$ the signal is deformed as shown in curve 62 of Fig. 7.

By means of the two capacitors 24 connected in series and providing, for example, a total capacity of C2 = 62.5 nF, the pulse applied by the ablation electrode 15 has the desired form shown in curve 63 in Fig. 8. In case the tissue impedance R2 changes, e.g. is reduced, the capacity of the catheter may be adapted in that, for example, one of the two capacitors 24 is deactivated or one or both capacitors may be adjustable and the total compensation capacity provided by the assembly 20 is greater.

The compensation impedance may be calculated with regard to the embodiment as follows. For calculation it is assumed that a desired pulse form has a predefined decay time $\tau$.

By using the formula

$$(1) \quad \tau = R \cdot C$$

The total capacity of two capacitors is calculated using formula

$$(2) \quad C_{ges} = \frac{C_1 \cdot C_2}{C_1 + C_2}$$

If $\tau$ is kept constant, a change of tissue impedance from $R_{low}$ to $R_{high}$ the total capacity needed is

$$(3) \quad C_{high\,R} = C_{low\,R} \cdot \frac{R_{low}}{R_{high}} ,$$

wherein $C_{low\,R}$ is the impedance $C1$ of the generator 30.

Accordingly, compensation capacity $C_{comp}$ (corresponds to C2 in Fig. 3) provided by assembly 20 (e.g. by means of capacitors 24) is

$$(4) \quad C_{comp} = \frac{C_{low\,R}}{\frac{R_{high}}{R_{low}} - 1} .$$

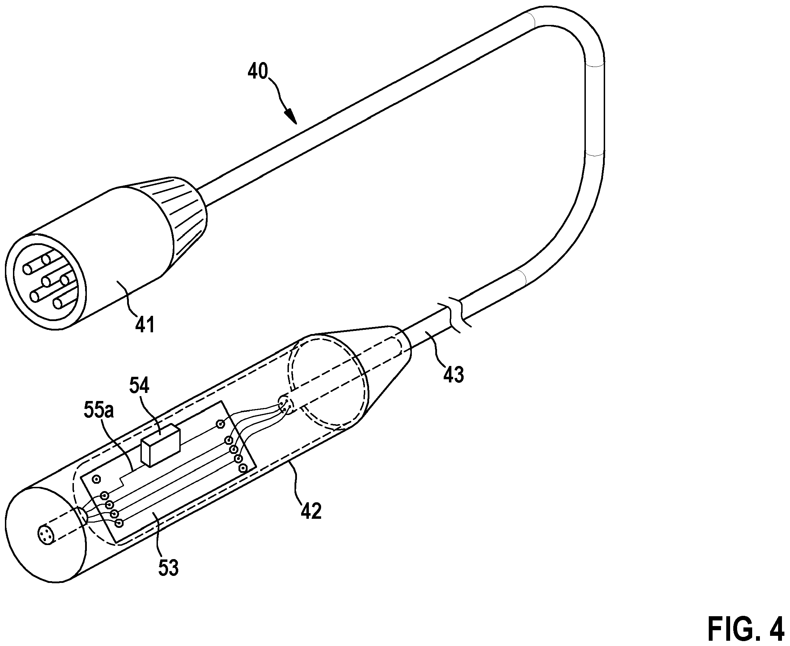

With regard to Fig. 4 to 6 another embodiment of a system is described having the power supply generator 30 analogous to the one of the system shown in Fig. 1 to 3. Further, an ablation catheter 10' (see Fig. 7) is used that does not have an assembly 20 but is otherwise similar to the ablation catheter 10 of Fig. 1.

Further, the system comprises a coupling device 40 comprising a first connector (plug) 41 and a second connector (socket) 42 that is configured to be interposed between the generator 30 and the catheter 10'. For that, the first connector 41 is electrically and mechanically coupled to the generator connector (not shown, socket) of the generator 30 and the second connector 42 to the catheter connector 19 (plug) of the ablation catheter 10'. Further, an isolating cable 43 is provided electrically connecting the lead pins of the first connector 41 to the lead pins of the second connector 42.

Within the second connector 42 an assembly 50 having an adjustable capacitor 54 is provided that is connected in series with the assembly input 51 and the assembly output 52 that is configured to be connected to the lead 55a for the ablation electrode. The signal/power supply of the ring electrodes 17 is provided by leads 57a.

The coupling device 40 is configured such that it is interposed between the generator 30 and the ablation catheter 10', wherein the assembly input 51 is electrically connected to the generator 30 output and the assembly output 52 is electrically connected to the ablation electrode input. The assembly 50 may be located at a PCB 53. The assembly 50 further comprises a capacitor 54 being, for example, a variable capacitance diode that is set by the HCP. The compensation of a tissue impedance of 100 $\Omega$ may be provided by a capacitance of 62.5 nF. The capacity may be set using a respective control element at the outer surface of the connector 42 housing.

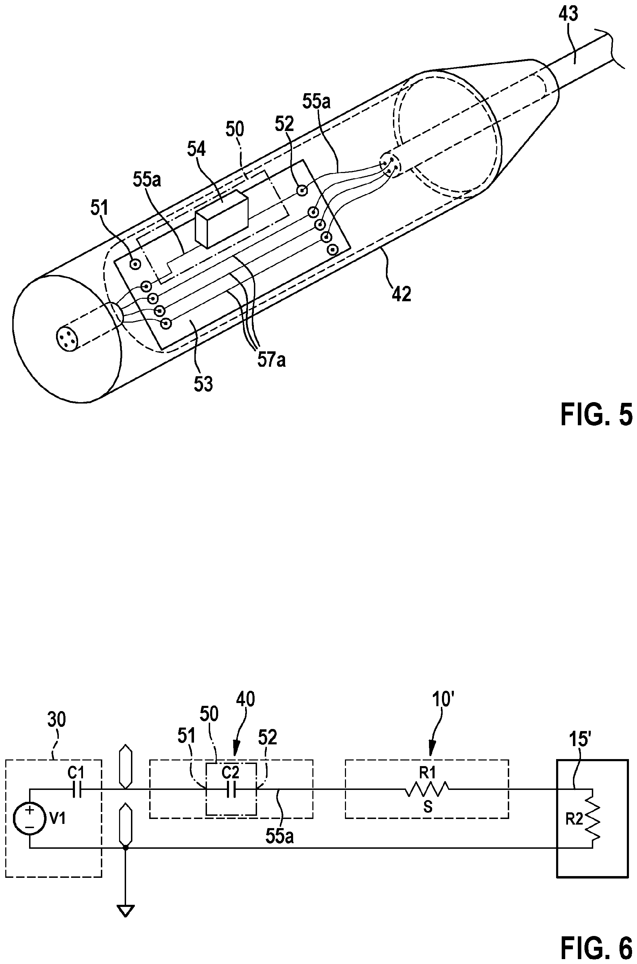

The electrical circuit for the system comprising the coupling device 40 is shown in Fig. 6. It is shown by this circuit, that the assembly 50 is electrically integrated within the coupling device 40. The coupling device 40 is interposed between the generator 30 and the ablation catheter 10'. With regard to the calculation of the compensating capacity C2 and the signal form, the situation is similar to the system of Fig. 1 to 3, when the electrical connections to the separate coupling device 40 are provided as shown in Fig. 6. It is therefore referred to the above explanation.

As indicated above, an impedance change from $R_{low}$ to $R_{high}$ may easily be compensated by an assembly 20, 50 having a capacitor unit with capacitors 24, 54 provided by the catheter 10 or by the separate coupling device 40. Accordingly, adaption of the power supply generator 30 is not necessary. Additionally, such assembly 20, 50 provides the conversion of monophasic pulses (provided by the generator 30) to biphasic pulses as shown in curve 63 in Fig. 8 that is applied at the ablation tip electrode 15. Additionally, the coupling device 40 may switch ablation mode between PFA and RF ablation.

**Claims**

1. An assembly (20, 50) for a system comprising a power supply generator (30) having a generator output and a catheter (10, 10') for treatment of a patient's tissue using an electrical field with at least one ablation electrode (15, 15'), each ablation electrode (15, 15') having an ablation electrode input, wherein the assembly (20, 50) comprises a capacitor unit, an assembly input and an assembly output, wherein the capacitor unit comprises at least one capacitor (24, 54) having a predefined capacity and is electrically connected to the assembly input (21, 51) and the assembly output (22, 52), wherein the assembly (20, 50) is configured to be interposed between and electrically connected to the generator output and the ablation electrode input of a predefined ablation electrode (15, 15').

2. The assembly of claim 1, wherein the capacitor unit comprises at least two capacitors that (24) are electrically connected in series.

3. The assembly of any one of the previous claims, wherein the capacitor unit comprises a parallel connection of at least two capacitors.

4. The assembly of any one of the previous claims, wherein the assembly further comprises an adjustable control element, wherein the control element is configured to control the capacity of an electrically adjustable capacitor according to the setting of the control element, and/or in case the capacitor unit comprises at least two capacitors, the control element is configured to activate a predefined capacitor or predefined capacitors of the at least two capacitors and a predefined interconnection of the activated capacitor(s) according to the setting of the control element.

5. The assembly of claim 4, wherein the assembly comprises a measuring unit configured to measuring the actual impedance of the tissue of the target location.

6. The assembly of any one of the previous claims, wherein the assembly comprises a switch unit for switching between at least two ablation modes, wherein the switch unit has a first switch unit input, a second switch unit input and a first switch unit output, wherein the first switch unit output is electrically connected to an input of capacitor unit, wherein the first switch unit input is electrically connected to the assembly input, wherein the second switch unit input is configured to receive a control signal from the power supply generator or from adjustment element.

7. A catheter (10) for treatment of a patient's tissue using an electrical field with at least one ablation electrode (15), each electrode having an ablation electrode input, wherein the catheter (10) further comprises a catheter connector (19) for electrical connection to a generator output of a power supply generator (30), wherein the catheter (10) comprises at least one assembly (20) of any one of the previous claims, wherein each assembly (20) is formed integrally with the catheter (10) such that the respective assembly input (21) is electrically connected to the catheter connector (19) and the respective assembly output (22) is electrically connected to the ablation electrode input of a predefined electrode (15).

8. The catheter of claim 7, wherein the catheter (10) comprises a supply line (18) connecting a handle (11) and the catheter connector (19), wherein each assembly is formed integrally with the supply line and/or with the catheter connector.

9. The catheter of any one of the claims 7 to 8, wherein each assembly (20) is formed integrally with the handle (11) of the catheter (10).

10. A coupling device (40) for a system comprising a power supply generator (30) having a generator connector and a catheter (10') for treatment of a patient's tissue using an electrical field with at least one ablation electrode, wherein the catheter comprises a catheter connector, wherein the coupling device (40) comprises an assembly (50) of any one of the claims 1 to 6, wherein the assembly (50) is integrally formed with the coupling device (40), wherein the coupling device (40) is configured to be electrically connected and detachably mechanically connected to the catheter connector and to the generator connector thereby providing an electrical connection from a generator output of the power supply generator (30) to an ablation electrode input via the assembly (50).

11. The coupling device of claim 10, wherein the coupling device comprises a box containing the assembly.

12. A system comprising a power supply generator (30) having a generator connector and a catheter (10') for treatment of a patient's tissue using an electrical field with at least one ablation electrode, wherein the catheter comprises a catheter connector, wherein the system further comprises a coupling device (40) formed separately from the catheter and from the generator, wherein the coupling device (40) comprises an assembly (50) of any one of the claims 1 to 6, wherein the assembly (50) is integrally formed with the coupling device (40), wherein the coupling device (40) is configured to be electrically connected to the catheter connector and to the generator connector thereby providing an electrical connection from a generator output of the power supply generator (30) to an ablation electrode input via the assembly (50).

13. The system of claim 12, wherein the coupling device comprises a box housing the assembly.

14. The system of any one of the claims 12 to 13, wherein the generator comprises a pulse-shaping output stage for coupling to the at least one ablation electrode.

15. The system of any one of the claims 12 to 14, wherein the generator comprises a high-voltage source, wherein the high-voltage source is configured to be set by the generator to provide a high-voltage amplitude of at least 1.000 V.

FIG. 1

FIG. 2

FIG. 3

FIG. 4

EP 4 591 814 A1

FIG. 5

FIG. 6

FIG. 7

FIG. 8

EP 4 591 814 A1

Europäisches
Patentamt

European
Patent Office

Office européen
des brevets

# EUROPEAN SEARCH REPORT

Application Number

EP 24 15 3333

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/200893 A1 (STRONG MARK [US]) 29 June 2023 (2023-06-29) * paragraphs [0002], [0024], [0033], [0051]; figure 1 * | 1-15 | INV. A61B18/12 A61B18/14 |
| X | CN 115 778 520 A (UNIV ZHENGZHOU) 14 March 2023 (2023-03-14) * the whole document * | 1,4,6,7, 10,12, 14,15 | ADD. A61B18/00 |
| X | US 2020/085498 A1 (XIAO SHU [US] ET AL) 19 March 2020 (2020-03-19) * paragraphs [0002], [0061], [0094] * | 1-6,10, 11 | |

TECHNICAL FIELDS
SEARCHED      (IPC)

A61B

The present search report has been drawn up for all claims

| Place of search | Date of completion of the search | Examiner |
|---|---|---|
| Munich | 13 June 2024 | Aronsson, Fredrik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT**

**ON EUROPEAN PATENT APPLICATION NO.**                EP 24 15 3333

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2024

| Patent document cited in search report | Publication date | Patent family member(s) | Publication date |
|---|---|---|---|
| US 2023200893 A1 | 29-06-2023 | EP 4125663 A1 | 08-02-2023 |
| | | JP 7482262 B2 | 13-05-2024 |
| | | JP 2023529146 A | 07-07-2023 |
| | | US 2023200893 A1 | 29-06-2023 |
| | | WO 2021247738 A1 | 09-12-2021 |
| CN 115778520 A | 14-03-2023 | NONE | |
| US 2020085498 A1 | 19-03-2020 | AU 2017225297 A1 | 23-08-2018 |
| | | CA 3015756 A1 | 08-09-2017 |
| | | CA 3113263 A1 | 08-09-2017 |
| | | CA 3113297 A1 | 08-09-2017 |
| | | CN 108702145 A | 23-10-2018 |
| | | CN 116800228 A | 22-09-2023 |
| | | EP 3424146 A1 | 09-01-2019 |
| | | ES 2866927 T3 | 20-10-2021 |
| | | IL 261128 A | 31-10-2018 |
| | | IL 291403 A | 01-05-2022 |
| | | JP 6706350 B2 | 03-06-2020 |
| | | JP 6924301 B2 | 25-08-2021 |
| | | JP 2019510597 A | 18-04-2019 |
| | | JP 2020127799 A | 27-08-2020 |
| | | JP 2021177775 A | 18-11-2021 |
| | | KR 20180107168 A | 01-10-2018 |
| | | KR 20210018539 A | 17-02-2021 |
| | | PL 3424146 T3 | 11-10-2021 |
| | | US 2017245928 A1 | 31-08-2017 |
| | | US 2020085498 A1 | 19-03-2020 |
| | | US 2021196375 A1 | 01-07-2021 |
| | | US 2023293231 A1 | 21-09-2023 |
| | | WO 2017151261 A1 | 08-09-2017 |

EPO FORM P0459

For more details about this annex : see Official Journal of the European Patent Office, No. 12/82